# EUROPEAN PATENT APPLICATION

(11) **EP 4 407 265 A1**
(43) Date of publication of application: **31.07.2024**
(21) Application number: 22871500.9
(22) Date of filing: 20.06.2022
(51) Int. Cl.: F25D 29/00, F25D 11/02, G01N 33/04

(54) **MICROFLUIDIC DETECTION SYSTEM FOR USE IN REFRIGERATOR, AND REFRIGERATOR**

(30) Priority: 23.09.2021 CN 202111115637
(71) Applicant: Qingdao Haier Refrigerator Co., Ltd., Laoshan District Qingdao Shandong 266101 (CN); Haier Smart Home Co., Ltd., Qingdao, Shandong 266101 (CN)
(72) Inventor: FEI, Bin, Qingdao, Shandong 266101 (CN); ZHAO, Bintang, Qingdao, Shandong 266101 (CN); ZHU, Xiaobing, Qingdao, Shandong 266101 (CN); LI, Mengcheng, Qingdao, Shandong 266101 (CN)
(74) Representative: Winter, Brandl - Partnerschaft mbB
(86) International application number: PCT/CN2022/099818
(87) International publication number: WO 2023/045439

(57) **Abstract**

A microfluidic control detection system and refrigerator, where the system comprises: a microfluidic biochip with an inlet, a suction port, and a detection pool formed inside, interconnected sequentially through microchannels, with the suction port located on a side surface parallel to width and length directions of the microfluidic biochip; a sample liquid driving device, configured to communicate with the suction port of the microfluidic biochip via a sealing connector once the biochip is in its installed position, to prompt the entry of the sample liquid in contact with the inlet into the microchannels and flow towards the detection pool; a pressing mechanism, configured to apply pressure perpendicular to the side surface to the sealing connector after the microfluidic biochip is placed in its position, to form a fluidic seal connection between the sealing connector and the suction port; and a detection mechanism for detecting the detection pool to obtain preset detection parameters of the sample liquid.

## Description

### CROSS-REFERENCE TO RELATED APPLICATION

The application claims priority from Chinese Patent Application No. 202111115637.1, filed September 23, 2021, entitled " Microfluidic Control Detection System and Refrigerator", all of which are incorporated herein by reference in their entirety.

### TECHNICAL FIELD

The present application relates to the field of refrigeration technology, and more particularly to a microfluidic control detection system and refrigerator.

### BACKGROUND

With the improvement of living standards, there is often a need in daily life to detect residues, viruses, nutritional elements, or other aspects of food ingredients either qualitatively or quantitatively. For instance, due to the misuse of pesticides, the fruits, vegetables, and agricultural products we buy daily may contain excessive levels of residues. If the excessive levels of residues in these foods are not discovered in time, ingestion can cause significant harm to the human body. Moreover, the currently advocated breastfeeding is best for infants only when the breast milk has normal nutritional value. However, illnesses, medication, surgery, or other situations of the nursing mother may lead to reduced nutritional element content in the secreted milk or even the presence of viruses, thereby affecting the infant's growth and health. However, the functionality of existing household appliances is relatively singular, and when there is a need to detect residues, viruses, nutritional elements, or other aspects of food ingredients, it necessitates the separate purchase of a dedicated detection device. This leads to a multitude of household appliances and types, occupying significant space, and does not align with the development trend of smart homes.

Among many detection methods, the use of microfluidic biochips for detection is relatively quick and compact, suitable for home use. To facilitate fluid movement within the chip, there are generally two methods: air pressure propulsion and centrifugal force propulsion. Centrifugal force propulsion relies on rotating centrifugal force to move droplets, which can only adjust unidirectional flow actions by adjusting the rotation speed. Air pressure propulsion utilizes positive air pressure and negative air pressure to bidirectionally propel fluid movement within the chip, offering high precision and controllability. However, air pressure propulsion needs to address the challenge of sealing the air pressure pipeline with the chip's suction port. Existing microfluidic detection systems mostly position the chip's suction port on its end surface to facilitate sealing the suction port with the air pressure pipeline upon chip installation. However, due to the thin thickness and small volume of the microfluidic biochip, the area of its end surface is very limited, restricting the size of the sealing interface between the air pressure pipeline and the suction port. This results in poor sealing between the air pressure pipeline and the suction port, easily leading to liquid leakage, air leakage, and imprecise control issues.

### SUMMARY

An object of a first aspect of the present application is to overcome at least one deficiency of the existing technology by providing a microfluidic control detection system for refrigerators that offers improved sealing performance and precise sample introduction control.

Another object of a first aspect of the present application is to simplify the detection operations and enhance user experience.

An object of a second aspect of the present application is to provide a refrigerator equipped with the aforementioned microfluidic control detection system.

In accordance with a first aspect of the present application, the present application provides a microfluidic control detection system for a refrigerator, comprising:
a microfluidic biochip, comprising an inlet, a suction port, and a detection pool formed inside, where the inlet, the detection pool, and the suction port are sequentially interconnected through microchannels, with the suction port located on a side surface parallel to a width direction and a length direction of the microfluidic biochip;
a sample liquid driving device, configured to be in communication with the suction port of the microfluidic biochip via a sealing connector after the microfluidic biochip is in its installed position, to prompt sample liquid in contact with the inlet to enter the microchannels and flow towards the detection pool under the control of the microfluidic biochip;
a pressing mechanism, configured to apply pressure perpendicular to the side surface to the sealing connector after the microfluidic biochip is installed in its position, to form a fluidic seal connection between the sealing connector and the suction port; and
a detection mechanism, configured to detect the detection pool to obtain preset detection parameters of the sample liquid.

In an embodiment of the present application, an internal part of the sealing connector forms a through connection channel, a first end of the connection channel is in communication with the sample liquid driving device, and after the microfluidic biochip is installed in its position, a second end of the connection channel is in communication with the suction port of the microfluidic biochip.

In an embodiment of the present application, the pressing mechanism comprises a lever rotating around a fixed rotation axis, comprising a first end and a second end;
the microfluidic biochip is configured to abut against the first end of the lever during installation, causing the lever to rotate around the rotation axis until the microfluidic biochip is installed in its position; and as the lever rotates around the rotation axis, the second end of the lever abuts against the sealing connector, prompting the sealing connector to move towards the side surface of the microfluidic biochip, thereby pressing the sealing connector against the side surface.

In an embodiment of the present application, the second end of the connection channel is provided with a gradually expanding part, cross-sectional area of the gradually expanding part gradually increases from inside outwards; and
after the microfluidic biochip is installed in its position, the gradually expanding part covers the suction port of the microfluidic biochip.

In an embodiment of the present application, the microfluidic control detection system further comprising:
a bracket, configured to support the sealing connector; and
the sealing connector comprises a cap part and a rod part that are perpendicular to each other, with the rod part being slidably inserted into the bracket to form a fluidic connection with the suction port; a spring is fitted outside the rod part, one end of the spring abuts against the inner side of the cap part towards the rod part, and another end of the spring abuts against the bracket; and the second end of the lever abuts against the outer side of the cap part away from the rod part.

In an embodiment of the present application, the sample liquid driving device is connected to a suction pipeline that is in fluidic connection with the sample liquid driving device; and
the cap part comprises a protruding connecting column inserted into the suction pipeline, allowing a connection channel inside the connecting column to be in sealed communication with the suction pipeline.

In an embodiment of the present application, the pressing mechanism comprises a spring operable to extend and retract in a predetermined direction, one end of the spring abuts against a fixed bracket, and another end of the spring abuts against the sealing connector; and
the spring is configured to be compressed by the pressure from the sealing connector moving away from the side surface during installation of the microfluidic biochip, and after the microfluidic biochip is installed in its position, the spring applies pressure to the sealing connector towards the side surface due to its elastic deformation recovery force, thereby pressing the sealing connector against the side surface.

In an embodiment of the present application, the microfluidic biochip further comprises a slot opened on the side surface;
the sealing connector comprises a body forming the connection channel inside and a first slanting push rod extending outward from the body; and
during the installation of the microfluidic biochip, the first slanting push rod abuts against the side surface and moves parallel to the side surface; and after the microfluidic biochip is installed in its position, the first slanting push rod is inserted into the slot, thereby securing the microfluidic biochip.

In an embodiment of the present application, the predetermined direction is parallel to an installation direction of the microfluidic biochip; and
a section of the body for cooperating with the suction port of the microfluidic biochip is perpendicular to the side surface, and the first slanting push rod extends from the section of the body perpendicular to the side surface in a direction inclined towards the side surface, so that interaction force between the microfluidic biochip and the sealing connector comprises component parallel to the side surface and component perpendicular to the side surface.

In an embodiment of the present application, the microfluidic control detection system further comprising:
a chip exit mechanism, configured to operatively apply a force in a direction away from the side surface to the sealing connector, thereby causing the first slanting push rod to detach from the slot.

In an embodiment of the present application, the sealing connector further comprises a second slanting push rod extending from the body in a direction inclined away from the side surface;
the chip exit mechanism comprises a pop-up button and an inclined chute for insertion of the second slanting push rod, and the pop-up button is configured to operatively move the inclined chute in a direction away from the side surface, thereby causing the second slanting push rod to have a displacement component moving away from the side surface.

In accordance with a second aspect of the present application, the present application provides a refrigerator, comprising the microfluidic control detection system according to any of the aforementioned technical solutions.

The microfluidic control detection system of the present application comprises a microfluidic biochip and a sample liquid driving device in communication with a suction port of the microfluidic biochip, utilizing air pressure to propel fluid movement within the microfluidic biochip. Specifically, the suction port of the microfluidic biochip is located on its side surface, parallel to a width direction and a length direction, which, compared to an end surface of the microfluidic biochip, offers a much larger area. This facilitates the formation of a relatively large sealing interface around the suction port, significantly improving sealing performance between the sample liquid driving device and the suction port, preventing liquid leakage, air leakage and enhancing the precision of sample introduction control by the sample liquid driving device.

Furthermore, the microfluidic control detection system of the present application is also equipped with a pressing mechanism. The pressing mechanism can apply pressure perpendicular to the side surface of the microfluidic biochip to a sealing connector located between the sample liquid driving device and the suction port of the microfluidic biochip after the microfluidic biochip is installed in its position. The pressure ensures that the sealing connector tightly presses against the side surface of the microfluidic biochip, further improving sealing performance of sealing interface formed between the sealing connector and the side surface around the suction port. Additionally, the pressing mechanism automatically applies pressure to the sealing connector upon installation of the microfluidic biochip, achieving coupling between chip installation and sealing connection. Apart from installing the chip, users do not need to perform any other actions, simplifying detection operations and enhancing user experience.

The present application integrates the microfluidic control detection system into a refrigerator, fully leveraging the storage function of the refrigerator to make a detection process more convenient and facilitating linked control between the microfluidic detection system and the refrigerator. This higher level of automation meets the needs of smart homes.

Further details and the advantages and features of the present application will become clearer to those skilled in art from the detailed description of the specific embodiments in conjunction with the accompanying drawings below.

### BRIEF DESCRIPTION OF THE DRAWINGS

The subsequent text will describe some specific embodiments of the present application in a detailed but non-limiting manner with reference to drawings. The same reference numerals in the drawings denote the same or similar parts or components. It should be understood by those skilled in the art that these drawings are not necessarily drawn to scale. In the drawings:
FIG. 1 shows a schematic structural diagram of a microfluidic control detection system for a refrigerator according to an embodiment of the present application.
FIG.2 shows a schematic structural diagram of internal structure of a microfluidic control detection system according to an embodiment of the present application.
FIG.3 shows a partial schematic diagram of a microfluidic control detection system according to an embodiment of the present application.
FIG.4 shows a schematic structural diagram of a microfluidic biochip according to an embodiment of the present application.
FIG.5 shows a schematic sectional view of part of structure of a microfluidic control detection system according to an embodiment of the present application.
FIG.6 shows a schematic enlarged view of Part A in FIG. 5.
FIG.7 shows a schematic structural diagram of a microfluidic biochip and its clamping mechanism according to an embodiment of the present application.
FIG.8 shows a partial schematic diagram of a microfluidic detection mechanism according to another embodiment of the present application.
FIG.9 shows a schematic sectional view of part of structure of a microfluidic detection mechanism according to another embodiment of the present application.
FIG. 10 shows a schematic structural diagram of a refrigerator according to an embodiment of the present application.

### DETAILED DESCRIPTION

The present application initially provides a microfluidic control detection system for a refrigerator. The microfluidic control detection system of the present application is configured for the qualitative or quantitative detection of preset detection parameters of sample liquids. These preset detection parameters could include parameters indicating whether the amount of pesticide residue exceeds standards and/or the specific numerical value of the pesticide residue, parameters indicating whether nutritional elements meet standards and/or the specific content of nutritional elements, parameters for indicating whether specific harmful substances (such as specific viruses) exceed standards and/or specific content of specific harmful substances, among others.

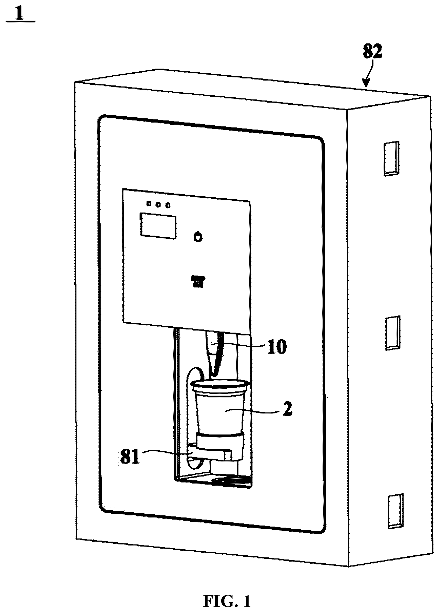

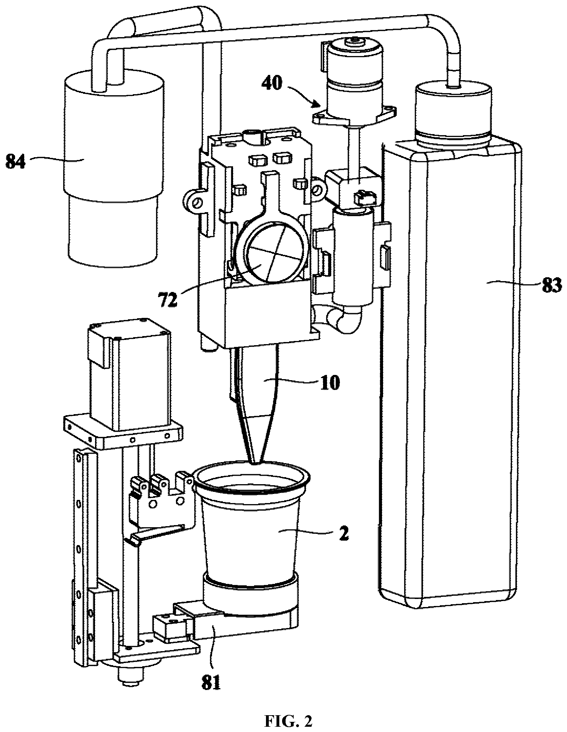

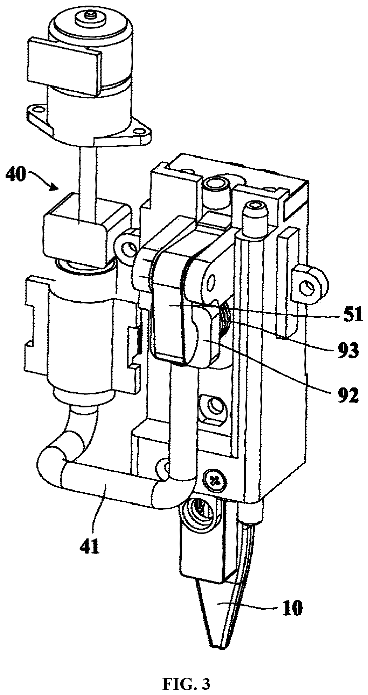

FIG. 1 shows a schematic structural diagram of a microfluidic control detection system for a refrigerator according to an embodiment of the present application, FIG.2 shows a schematic structural diagram of internal structure of a microfluidic control detection system according to an embodiment of the present application, and FIG.3 shows a partial schematic diagram of a microfluidic control detection system according to an embodiment of the present application. For ease of understanding, FIG. 1 and FIG.2 also show a sample cup 2.

Referring to FIG. 1 to FIG.3, the microfluidic control detection system 1 related to the present application comprises a microfluidic biochip 10, a sample liquid driving device 40, a pressing mechanism, and a detection mechanism 20.

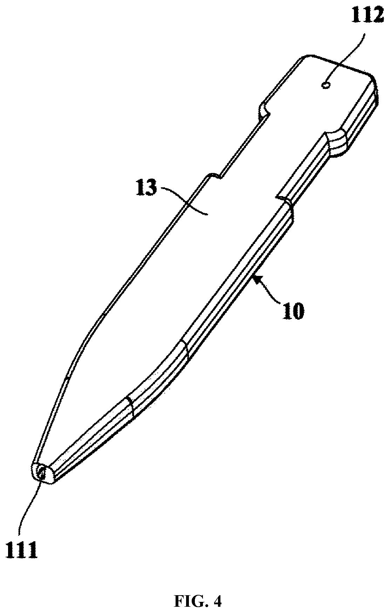

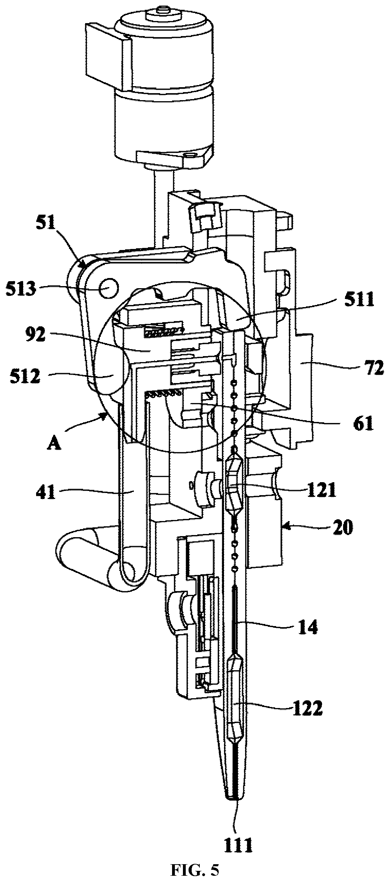

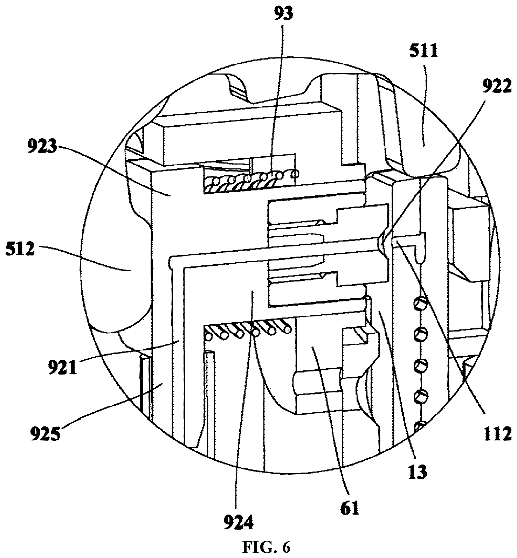

FIG.4 shows a schematic structural diagram of a microfluidic biochip according to an embodiment of the present application, FIG. 5 shows a schematic sectional view of part of the structure of a microfluidic control detection system according to an embodiment of the present application, and FIG.6 shows a schematic enlarged view of Part A in FIG.5. The microfluidic biochip 10 comprises an inlet 111, a suction port 112, and a detection pool 121 formed inside the microfluidic biochip 10. The inlet 111, detection pool 121, and suction port 112 are sequentially interconnected through microchannels 14, thus forming a main channel. The inlet 111 can be located on an end face of the microfluidic biochip 10 to facilitate contact with sample liquid. The suction port 112 is located on a side surface 13 that is parallel to the a width direction and a length direction of the microfluidic biochip 10. It can be understood that the microfluidic biochip 10 usually has a thin, flat shape, where the side surface 13 refers to a side surface parallel to both the width direction and the length direction of the microfluidic biochip 10, perpendicular to a thickness direction of the microfluidic biochip 10. The end face of the microfluidic biochip 10 refers to a surface located at an end of the microfluidic biochip 10. It can also be understood that the microchannels referred to in the present application are fine or capillary channels with a cross-sectional flow area within a predetermined size range, to ensure the microchannels have appropriate capacity to retain liquid inside.

The sample liquid driving device 40 is configured to communicate with the suction port 112 of the microfluidic biochip 10 via a sealing connector 92 once the microfluidic biochip 10 is in its installed position, thereby, under the control of the microfluidic biochip 10, prompting sample liquid in contact with the inlet 111 to enter the microchannels 14 and flow towards the detection pool 121. After the microfluidic biochip 10 is installed in its position, it means that the microfluidic biochip 10 has been installed at its designated location, i.e., installation of the microfluidic biochip 10 is complete.

The pressing mechanism is configured to apply pressure perpendicular to the side surface 13 to the sealing connector 92 after the installation of the microfluidic biochip 10 in its position, to form a fluidic seal connection between the sealing connector 92 and the suction port 112.

The detection mechanism 20 is configured to detect the detection pool 121 to obtain preset detection parameters of the sample liquid. Specifically, detection reagents can be pre-placed in the detection pool 121, or can be manually or automatically added to the detection pool 121, so that after a reaction of the sample liquid with the detection reagents inside the detection pool 121, the detection mechanism 20 performs detection on the detection pool 121.

The microfluidic control detection system 1 of the present application comprises a microfluidic biochip 10 and a sample liquid driving device 40 in communication with a suction port 112 of the microfluidic biochip 10, to utilize air pressure to drive movement of fluid within the microfluidic biochip 10. Specifically, the suction port 112 of the microfluidic biochip 10 is located on a side surface 13 parallel to a width direction and a length direction, which offers a much larger area compared to an end face of the microfluidic biochip 10. This is advantageous for forming a relatively large sealing interface around the suction port 112, thereby significantly improving sealing performance between the sample liquid driving device 40 and the suction port 112, preventing liquid leakage, air leakage and enhancing the precision of sample introduction control by the sample liquid driving device 40.

Additionally, the microfluidic control detection system 1 of the present application is also equipped with a pressing mechanism. The pressing mechanism can apply pressure perpendicular to the side surface 13 of the microfluidic biochip 10 to a sealing connector 92 located between the sample liquid driving device 40 and the suction port 112 of the microfluidic biochip 10 after the microfluidic biochip 10 is installed in its position. The pressure ensures the sealing connector 92 tightly presses against the side surface 13 of the microfluidic biochip 10, further improving sealing performance of sealing interface formed between the sealing connector 92 and the side surface around the suction port 112. Furthermore, the pressing mechanism automatically applies pressure to the sealing connector 92 upon the installation of the microfluidic biochip 10, achieving coupling between chip installation and sealing connection. Users do not need to perform any actions other than installing the chip, simplifying detection operations and enhancing user experience.

It is understandable to those skilled in art that when the microfluidic control detection system is used for detecting different preset detection parameters, the specific choices of the microfluidic biochip 10 and the detection mechanism 20 might also vary. For example, when the microfluidic control detection system is used for pesticide residue detection, the microfluidic biochip 10 it contains could be a microfluidic pesticide detection chip capable of providing conditions for pesticide liquid detection, and the detection mechanism 20 it contains could be a pesticide detection mechanism capable of detecting pesticide parameters in the pesticide liquid.

In a specific embodiment, when the detection mechanism 20 is a pesticide detection mechanism for detecting pesticide parameters in pesticide liquid, a quick qualitative detection of whether pesticide residue in sample liquid exceeds standards can be conducted using the enzyme inhibition rate method. In this case, the microfluidic biochip 10 further comprises a reaction pool 122 formed inside it. The reaction pool 122 is located on the main channel formed by sequentially connecting the inlet 111, the detection pool 121, and the connecting port 112 and is connected between the inlet 111 and the detection pool 121, allowing sample liquid to react with reaction reagents in the reaction pool 122 before flowing into the detection pool 121. The reaction pool 122 is connected to both the inlet 111 and the detection pool 121 through microchannels 14. The reaction reagent and detection reagent used for pesticide detection can be enzyme reagents and chromogenic agents, respectively. The reaction pool 122 is used for sample liquid to react with the enzyme reagent inside it; the sample liquid that has reacted with the enzyme reagent flows into the detection pool 121 and reacts with the chromogenic agent in the detection pool 121. The detection mechanism 20 can be selected as a photoelectric detection mechanism, which may comprise structures such as a light source, a photosensitive element, a heating plate, and a thermostat.

In some embodiments, an internal part of the sealing connector 92 forms a through connection channel 921. A first end of the connection channel 921 communicates with the sample liquid driving device 40, and after the microfluidic biochip 10 is installed in its position, a second end of the connection channel 921 communicates with the suction port 112 of the microfluidic biochip 10. Thus, the sample liquid driving device 40 can be in fluid communication with the suction port 112 of the microfluidic biochip 10 through the connection channel 921.

Furthermore, the second end of the connection channel 921 is provided with a gradually expanding part 922, whose cross-sectional area gradually increases from inside outwards, enabling the second end of the connection channel 921 to have a larger opening area. After the microfluidic biochip 10 is installed in its position, the gradually expanding part 922 covers the suction port 112 of the microfluidic biochip 10, thereby forming fluid communication between the connection channel 921 and the suction port 112. It is understandable that there may be positional deviations when installing the microfluidic biochip 10, and configuration of the expanding part 922 allows for certain positional offsets between the connection channel 921 and the suction port 112, thus ensuring stable fluid communication between the suction port 112 and the connection channel 921 within an allowable range of installation errors. Simultaneously, after the microfluidic biochip 10 is installed in its position, a relatively large sealing interface is formed between the sealing connector 92 and the side surface 13, resulting in better sealing performance.

In some embodiments, the pressing mechanism includes a lever 51 that rotates around a fixed rotation axis 513. The lever 51 comprises a first end 511 and a second end 512. The microfluidic biochip 10 is configured to abut against the first end 511 of the lever 51 during installation, causing the lever 51 to rotate around its rotation axis 513 until the microfluidic biochip 10 is installed in its position. As the lever 51 rotates around its rotation axis 513, the second end 512 of the lever 51 abuts against the sealing connector 92 and prompts the sealing connector 92 to move in a direction gradually approaching the side surface 13 of the microfluidic biochip 10, thereby pressing the sealing connector 92 against the side surface 13. Once the microfluidic biochip 10 is installed in its position, the lever 51 no longer rotates, and the second end 512 of the lever 51 maintains its abutting action against the sealing connector 92, thus keeping the sealing connector 92 pressed against the side surface 13.

Leveraging the principle of levers, the first end 511 and the second end 512 of the lever 51 can be positioned at different locations and can extend in different directions. Therefore, movement direction of the first end 511 of the lever15 and movement direction of the second end 512 of the lever 51 can be varied according to their extension directions. This enables the coupling of chip installation and sealing connection even if the installation direction of the microfluidic biochip 10 and the direction in which the sealing connector 92 presses against the side surface 13 of the microfluidic biochip 10 are perpendicular to each other. Additionally, structure of lever is very simple, not complicating structure of the microfluidic detection system 1.

In some embodiments, the microfluidic control detection system 1 also comprises a first bracket 61 configured to support the sealing connector 92. The sealing connector 92 comprises a cap part 923 and a rod part 924, which are perpendicular to each other. The rod part 924 can be slidably inserted into the first bracket 61 to form a fluid connection with the suction port 112. A first spring 93 is fitted around the exterior of the rod part 924, with one end of the first spring 93 abutting against the inner side of the cap part 923 towards the rod part 924, and another end of the first spring 94 abutting against the first bracket 61. The second end of the lever 51 abuts against the outer side of the cap part 923, away from the rod part 924.

Furthermore, the first end 511 of the lever 51 can be located near the installation position of the microfluidic biochip 10 to abut against an end face of the microfluidic biochip 10 during its installation process, and rotate as the microfluidic biochip 10 is installed. During the rotation of the lever 51, its second end 512 applies pressure towards the sealing connector 92 in the direction towards the microfluidic biochip 10, causing the first spring 93 to compress. When the microfluidic biochip 10 is being disassembled, abutting action of the microfluidic biochip 10 against the first end 511 of the lever decreases or disappears, under elastic deformation recovery force of the first spring 93, the sealing connector 92 applies a force on the second end 512 of the lever 51 in the direction away from the microfluidic biochip 10, causing the lever 51 to rotate in the opposite direction, and the sealing connector 92 to detach from the side surface 13 of the microfluidic biochip 10. Simultaneously, the first end 511 of the lever 51 can also generate a force that facilitates the popping out of the microfluidic biochip 10, aiding in smooth disassembly of the microfluidic biochip 10.

Specifically, the lever 51 can be roughly U-shaped.

In some embodiments, the sample liquid driving device 40 is connected to a suction pipeline 41 that is in fluid communication with it. The cap part 923 comprises a protruding extension in the form of a connecting column 925, which is inserted into the suction pipeline 41 to ensure that the internal connection channel 921 of the connecting column 925 is in sealed communication with the suction pipeline 41. Since the connecting column 925 is connected to the suction pipeline 41 through an insertion method, a large contact area between them ensures good sealing performance.

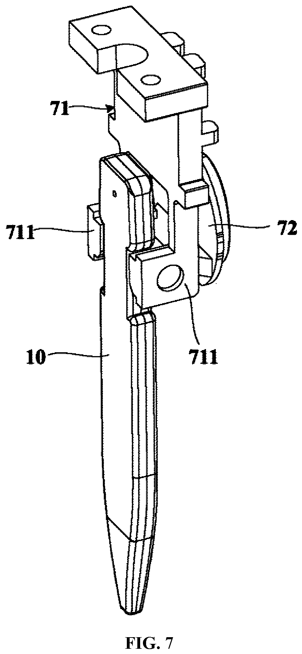

FIG.7 illustrates a schematic structure of a microfluidic biochip and its clamping mechanism according to an embodiment of the present application. In some embodiments, the microfluidic detection system 1 further comprises a clamping mechanism 71, which is configured to clamp the microfluidic biochip 10, ensuring it maintains a fluid-sealed connection with the sample liquid driving device 40. Specifically, the clamping mechanism 71 may consist of two oppositely positioned elastic claws 711, applying opposing forces to the microfluidic biochip 10 clamped between them.

Additionally, the microfluidic detection system 1 comprises a cantilevered push-button 72, suspended on one side of the microfluidic biochip 10. The cantilevered push-button 72 simultaneously contacts the inner sides of the two elastic claws 711, arranged to face each other. This design allows the cantilevered push-button 72 to exert an outward force on the inner sides of the two elastic claws 711 when a force is applied to the cantilevered push-button 72 in the direction towards the microfluidic biochip 10, causing the two elastic claws 711 to elastically deform outwardly away from each other. In other words, to disassemble the microfluidic biochip 10, a user simply needs to press the cantilevered push-button to release clamping action of the two elastic claws 711 on the microfluidic biochip 10, thus freeing the microfluidic biochip 10. This operation is straightforward, and structure of the cantilevered push-button 72 is ingeniously simple.

More specifically, the microfluidic biochip 10 can be installed in its position by moving upwards in a vertical direction, with the inlet 111 located at the bottom of the microfluidic biochip 10. The cantilevered push-button 72 is located on the front side of the microfluidic biochip 10 for easy pressing by the user. The suction port 112 is located on the side surface 13 at the back of the microfluidic biochip 10, with the sealing connector 92 positioned behind the microfluidic biochip 10. The first end 511 of the lever 51 is located above the microfluidic biochip 10, contacting top end of the microfluidic biochip 10, while the second end 512 of the lever 51 contacts a rear surface of the sealing connector 92, applying a forward force on it.

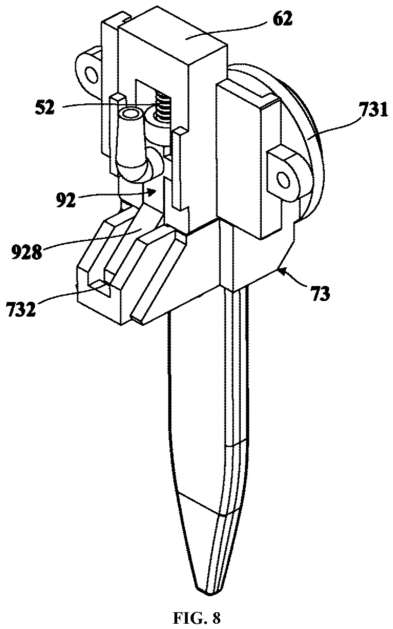

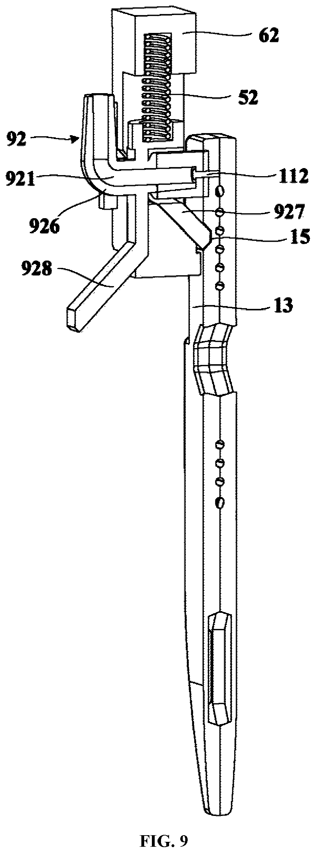

FIG.8 depicts a partial schematic diagram of a microfluidic detection mechanism according to another embodiment of the present application, and FIG.9 depicts schematic diagram of a portion of a microfluidic detection mechanism according to another embodiment of the present application. In other embodiments, the pressing mechanism comprises a second spring 52 that is operable to extend and retract along a predetermined direction. One end of the second spring 52 abuts against a fixed second bracket 62, while another end of the second spring 52 abuts against the sealing connector 92. The second spring 52 is configured to be compressed under the pressure from the sealing connector 92 moving away from the side surface 13 during installation of the microfluidic biochip 10. After the microfluidic biochip 10 is installed in its position, the second spring 52, under its own elastic deformation recovery force, applies pressure towards the side surface 13 to the sealing connector 92, thus pressing the sealing connector 92 against the side surface 13. It's understood that structure of the sealing connector 92 in embodiments shown in FIG. 8 and FIG.9 slightly differs from structure of the sealing connector 92 in embodiments shown in FIG.3 to FIG.7.

Further, the microfluidic biochip 10 comprises a slot 15 opened on the side surface 13. In these embodiments, the sealing connector 92 comprises a body 926, within which a connection channel 921 is formed, and a first slanting push rod 927 extending outward from the body 926. During the installation of the microfluidic biochip 10, the first slanting push rod 927 abuts against the side surface 13 and moves parallel to the side surface 13. Once the microfluidic biochip 10 is installed in its position, the first slanting push rod 927 inserts into the slot 15, thereby securing the microfluidic biochip 10.

This means, in some embodiments, an objective of holding the microfluidic biochip 10 is achieved by providing a slot 15 on the side surface 13 of the microfluidic biochip 10, incorporating the first slanting push rod 927 on the sealing connector 92, and through the interaction between the first slanting push rod 927 and the slot 15, without a need for additional chip clamping mechanisms, simplifying structure of the microfluidic detection system 1.

In some embodiments, the predetermined direction is parallel to an installation direction of the microfluidic biochip 10. This means that the second spring 52 extends and contracts along the installation direction of the microfluidic biochip 10. A section of the main 926, which is configured to cooperate with the suction port 112 of the microfluidic biochip 10, is perpendicular to the side surface 13. The first slanting push rod 927 extends from the section of the body 926, which is perpendicular to the side surface 13, inclined towards the side surface 13. This arrangement ensures that interaction force exerted between the microfluidic biochip 10 and the sealing connector 92 comprises component parallel to the side surface 13 and component perpendicular to the side surface 13. In other words, during the installation of the microfluidic biochip 10, the first slanting push rod 927 abuts against the side surface 13, and force generated by the microfluidic biochip 10 on the sealing connector 92 include components both parallel and perpendicular to the side surface 13. The parallel component compresses the second spring 52, while the perpendicular component causes a slight tilting deformation of the second spring 52. Once the microfluidic biochip 10 is installed, the first slanting push rod 927 aligns with the slot 15, and under the elastic deformation recovery force of the second spring 52, the second spring 52 exerts forces on the sealing connector 92 both parallel and perpendicular to the side surface 13. This not only enables the first slanting push rod 927 to engage with the slot 15 but also causes the second spring 52 to exert a component perpendicular to the side surface 13 on the sealing connector 92, pressing the sealing connector 92 against the side surface 13 and ensuring a sealed connection between the internal connection channel 921 of the sealing connector 92 and the suction port 112.

Additionally, in some embodiments, the microfluidic detection system 1 comprises a chip exit mechanism 73, configured to apply a force on the sealing connector 92 in a direction away from the side surface 13, facilitating the disengagement of the first slanting push rod 927 from the slot 15. This makes it easier for users to manually disassemble the microfluidic biochip 10.

Furthermore, a third spring can be positioned at an end face of the microfluidic biochip 10. After installation of the microfluidic biochip 10, this third spring is compressed; upon the disengagement of the first slanting push rod 927 from the slot 15, the third spring automatically ejects the microfluidic biochip 10, simplifying the operation for the user.

In some embodiments, the sealing connector 92 further comprises a second slanting push rod 928 extending from the body 926 in a direction inclined away from the side surface 13. The chip exit mechanism 73 is equipped with a pop-up button 731 and an inclined chute 732 for insertion of the second slanting push rod 928. The pop-up button 731 is configured to be operable to move the inclined chute 732 in a direction away from the side surface 13, thereby enabling the second slanting push rod 928 to have a displacement component moving away from the side surface 13. This allows users to simply press the push button 731 to disengage the first slanting push rod 927 from the slot 15, simplifying the operation.

In some embodiments, the sample liquid driving device 40 may be a micro injection pump that creates a vacuum within a main channel by drawing air outwards, thereby allowing sample liquid in contact with the inlet port 111 to enter the main channel under the influence of the vacuum. Specifically, the sample liquid driving device 40 comprises a drive motor, a vertically extending syringe, a lead screw, a slider, and a piston, among other components.

In some embodiments, the microfluidic biochip 10 also comprises a sample platform81. The sample platform 81 is positioned below the microfluidic biochip 10 for placing a sample cup 2, which holds the sample liquid. Furthermore, the sample platform 81 is configured to move in a controlled or operable manner to transport the sample cup 2 placed on the sample platform 81 to a position where sample liquid within the sample cup 2 is allowed to contact the inlet port 111 of the microfluidic biochip 10.

In some embodiments, the microfluidic detection system 1 may comprise a housing 82. The housing 82 has an operation platform open towards front side of the housing 82, with the sample platform 81 at least partially located within the operation platform, facilitating user operations such as placing the sample cup 2 or removing the sample cup 2.

In some embodiments, the microfluidic detection system 1 may comprise a buffer solution bottle 83 and a buffer solution driving device 84. The buffer solution bottle 83, located within the housing 82, is configured to contain a buffer solution. The buffer solution driving device 84, also located within the housing 82 and connected to the buffer solution bottle 83, is controlled to drive the buffer solution from the buffer solution bottle 83 into a sample cup 2 on the sample platform 81. Mixture of buffer solution and sample in the sample cup 2 produces sample liquid. Specifically, the buffer solution driving device 84 can be a peristaltic pump, diaphragm pump, or another suitable type of driving device.

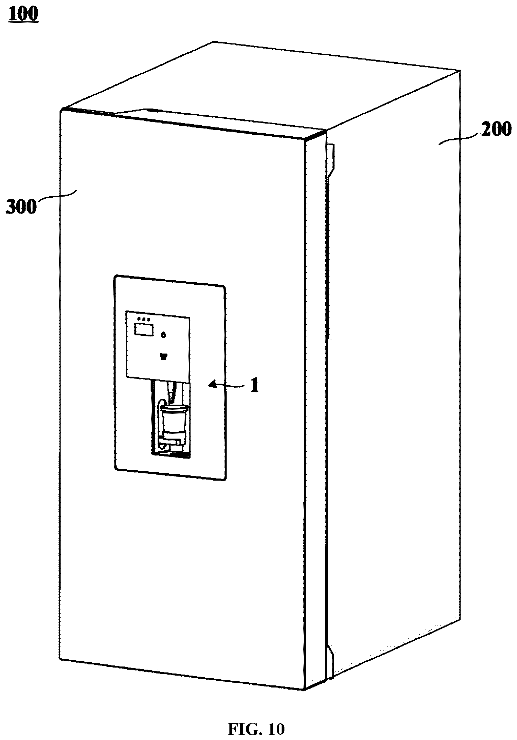

The present application also introduces a refrigerator, as illustrated in FIG. 10, illustrating a schematic structural diagram of a refrigerator according to an embodiment of the present application. The refrigerator 100 incorporates the microfluidic detection system 1 involved in any of the aforementioned embodiments, integrating the microfluidic detection system 1 with the refrigerator 100. Given the high frequency of use of refrigerators 100 in daily life and their primary function for storing food ingredients, integrating the microfluidic detection system 1 into the refrigerator 100 facilitates users in conducting detection operations on food ingredient samples using the microfluidic detection system 1.

By integrating the microfluidic detection system 1 into the refrigerator 100, the present application fully leverages storage function of the refrigerator 100, making a detection process more convenient. It also facilitates the interlinked control between the microfluidic detection system 1 and the refrigerator 100, achieving a high level of intelligence that meets needs of smart homes.

Additionally, the refrigerator 100 comprises a cabinet 200 and a door 300. The cabinet 200 defines a storage space, and the door 300 is connected to the cabinet 200 to open and/or close the storage space. Preferably, the microfluidic detection system 1 is mounted on the door 300, which is not only convenient for operation but also does not occupy an original storage space inside the cabinet 200, thus not affecting storage capacity of the refrigerator 100 itself.

The refrigerator 100 mentioned in the present application is broadly defined to include not only the conventional narrow sense of refrigerators but also storage devices with refrigeration, freezing, or other storage functions, such as refrigeration boxes, freezers, etc.

It should be understood by those skilled in the art that the terms "up," "down," "front," "back," "top," "bottom," and other directional or positional terms used in the embodiments of the present application are based on an actual usage state of the microfluidic detection system 1 and the refrigerator 100. These terms are merely for the convenience of describing and understanding the technical solutions of the present application and should not be interpreted as limiting the present application to specific orientations or configurations.

Thus, it should be recognized by those skilled in art that although this document has thoroughly presented and described several exemplary embodiments of the present application, many other variations or modifications that are in accordance with the principles of the present application can be directly determined or derived from the content disclosed here without departing from the spirit and scope of the present application. Therefore, the scope of the present application should be understood and recognized to cover all such other variations or modifications.

## Claims

1. A microfluidic control detection system for a refrigerator, **characterized in that** the microfluidic control detection system comprises:
a microfluidic biochip, comprising an inlet, a suction port, and a detection pool formed inside, where the inlet, the detection pool, and the suction port are sequentially interconnected through microchannels, with the suction port located on a side surface parallel to a width direction and a length direction of the microfluidic biochip;
a sample liquid driving device, configured to be in communication with the suction port of the microfluidic biochip via a sealing connector after the microfluidic biochip is in its installed position, to prompt sample liquid in contact with the inlet to enter the microchannels and flow towards the detection pool under the control of the microfluidic biochip;
a pressing mechanism, configured to apply pressure perpendicular to the side surface to the sealing connector after the microfluidic biochip is installed in its position, to form a fluidic seal connection between the sealing connector and the suction port; and
a detection mechanism, configured to detect the detection pool to obtain preset detection parameters of the sample liquid.

2. The microfluidic control detection system according to claim 1, **characterized in that**:
an internal part of the sealing connector forms a through connection channel, a first end of the connection channel is in communication with the sample liquid driving device, and after the microfluidic biochip is installed in its position, a second end of the connection channel is in communication with the suction port of the microfluidic biochip.

3. The microfluidic control detection system according to claim 2, **characterized in that**:
the pressing mechanism comprises a lever rotating around a fixed rotation axis, comprising a first end and a second end; the microfluidic biochip is configured to abut against the first end of the lever during installation, causing the lever to rotate around the rotation axis until the microfluidic biochip is installed in its position; and as the lever rotates around the rotation axis, the second end of the lever abuts against the sealing connector, prompting the sealing connector to move towards the side surface of the microfluidic biochip, thereby pressing the sealing connector against the side surface.

4. The microfluidic control detection system according to claim 3, **characterized in that**:
the second end of the connection channel is provided with a gradually expanding part, cross-sectional area of the gradually expanding part gradually increases from inside outwards; and
after the microfluidic biochip is installed in its position, the gradually expanding part covers the suction port of the microfluidic biochip.

5. The microfluidic control detection system according to claim 3 or 4, **characterized in that** the microfluidic control detection system further comprises:
a bracket, configured to support the sealing connector; and
the sealing connector comprises a cap part and a rod part that are perpendicular to each other, with the rod part being slidably inserted into the bracket to form a fluidic connection with the suction port; a spring is fitted outside the rod part, one end of the spring abuts against the inner side of the cap part towards the rod part, and another end of the spring abuts against the bracket; and the second end of the lever abuts against the outer side of the cap part away from the rod part.

6. The microfluidic control detection system according to claim 5, **characterized in that**:
the sample liquid driving device is connected to a suction pipeline that is in fluidic connection with the sample liquid driving device; and
the cap part comprises a protruding connecting column inserted into the suction pipeline, allowing a connection channel inside the connecting column to be in sealed communication with the suction pipeline.

7. The microfluidic control detection system according to claim 2, **characterized in that**:
the pressing mechanism comprises a spring operable to extend and retract in a predetermined direction, one end of the spring abuts against a fixed bracket, and another end of the spring abuts against the sealing connector; and
the spring is configured to be compressed by the pressure from the sealing connector moving away from the side surface during installation of the microfluidic biochip, and after the microfluidic biochip is installed in its position, the spring applies pressure to the sealing connector towards the side surface due to its elastic deformation recovery force, thereby pressing the sealing connector against the side surface.

8. The microfluidic control detection system according to claim 7, **characterized in that**:
the microfluidic biochip further comprises a slot opened on the side surface;
the sealing connector comprises a body forming the connection channel inside and a first slanting push rod extending outward from the body; and
during the installation of the microfluidic biochip, the first slanting push rod abuts against the side surface and moves parallel to the side surface; and after the microfluidic biochip is installed in its position, the first slanting push rod is inserted into the slot, thereby securing the microfluidic biochip.

9. The microfluidic control detection system according to claim 8, **characterized in that**:
the predetermined direction is parallel to an installation direction of the microfluidic biochip; and
a section of the body for cooperating with the suction port of the microfluidic biochip is perpendicular to the side surface, and the first slanting push rod extends from the section of the body perpendicular to the side surface in a direction inclined towards the side surface, so that interaction force between the microfluidic biochip and the sealing connector comprises component parallel to the side surface and component perpendicular to the side surface.

10. The microfluidic control detection system according to claim 8 or 9, **characterized in that** the microfluidic control detection system further comprises:
a chip exit mechanism, configured to operatively apply a force in a direction away from the side surface to the sealing connector, thereby causing the first slanting push rod to detach from the slot.

11. The microfluidic control detection system according to claim 10, **characterized in that**:
the sealing connector further comprises a second slanting push rod extending from the body in a direction inclined away from the side surface;
the chip exit mechanism comprises a pop-up button and an inclined chute for insertion of the second slanting push rod, and the pop-up button is configured to operatively move the inclined chute in a direction away from the side surface, thereby causing the second slanting push rod to have a displacement component moving away from the side surface.

12. A refrigerator, **characterized in that** the refrigerator comprises the microfluidic control detection system according to any one of claims 1-11.
